Europäisches Patentamt

**European Patent Office**

**Office européen des brevets**

(19)

(11) Numéro de publication : **0 117 163**
**B1**

(12) **FASCICULE DE BREVET EUROPÉEN**

(45) Date de publication du fascicule du brevet :
**19.08.87**

(51) Int. Cl.⁴ : **A 61 K   9/02**, A 61 F   5/47

(21) Numéro de dépôt : **84400031.5**

(22) Date de dépôt : **06.01.84**

(54) Dispositif endo-utérin contraceptif et thérapeutique combinant le culvre, la progestérone naturelle et l'acide amino iso caproique.

(30) Priorité : **07.01.83 FR 8300302**

(43) Date de publication de la demande :
**29.08.84 Bulletin 84/35**

(45) Mention de la délivrance du brevet :
**19.08.87 Bulletin 87/34**

(84) Etats contractants désignés :
**AT BE CH DE GB IT LI LU NL SE**

(56) Documents cités :
**FR-A- 2 250 520**
**FR-A- 2 479 685**
**CHEMICAL ABSTRACTS, vol.88, no.25, 19 juin 1978,
page 156, no.183643w, Columbus, Ohio, US;
A.T.L.ANDRADE et al.: "Effects of epsilon-aminocaproic acid on fertility in the rabbit"**
**CHEMICAL ABSTRACTS, vol.83, no.9, 1 septembre
1975, page 57, no.71802n, Columbus, Ohio, US; S.T.
SHAW Jr. et al.: "Intrauterine medication with epsilon
aminocaproic acid"**

(73) Titulaire : **Levrier, Marc**
**24 avenue de Lattre-de-Tassigny**
**F-33400 Talence-Bordeaux (FR)**

(72) Inventeur : **Levrier, Marc**
**24 avenue de Lattre-de-Tassigny**
**F-33400 Talence-Bordeaux (FR)**

(74) Mandataire : **Portal, Gérard et al**
**Cabinet Z. Weinstein 20, Avenue de Friedland**
**F-75008 Paris (FR)**

Jouve, 18, rue St-Denis, 75001 Paris, France

## Description

La présente invention concerne essentiellement un dispositif endo utérin contraceptif et thérapeutique, encore dénommé dispositif intra utérin ou même stérilet, tel que décrit ci-dessous.

On connait déjà de nombreux dispositifs endo utérin contraceptifs. Par exemple la demande de brevet français 2 479 685 concerne un dispositif intra-utérin comportant un fil de cuivre ayant une âme résistant à la corrosion en métal flexible plus noble que le cuivre, spécialement l'argent.

La demande de brevet français 2 250 520 décrit un dispositif intra-utérin actif formé par un substrat hydrophobe de grande souplesse mécanique caractérisé en ce que le substrat comporte dans son volume des inclusions de corps hydrophiles polymérisés dans lesquelles ont été emmagasinés des agents solubles dans l'eau, lesdits agents perfusant à travers le substrat hydrophobe lorsque le substrat est placé en milieu aqueux (page 2, ligne 36 à page 3 ligne 2 et revendication 1). On prévoit selon ce document un nombre important d'actions possibles en fonction de la nature des agents chimiques inclus dans les inclusions de corps hydrophiles polymérisés, à savoir une action contraceptive, un traitement des métrites et endométrites par antibiotiques, un traitement du cancer de l'endomètre par hormonothérapie (progestatifs à haute dose), un traitement de la stérilité en incluant dans les inclusions hydrophiles un produit tampon à pH contrôlé, un traitement de la ménopause par les progestérones, un traitement des synéchies, tous autres traitements de l'appareil génital de la femme (fibromes, kystes etc), traitement de la tuberculose génitale par la cortisone, avortement thérapeutique par la prostaglandine.

Ainsi, un nombre considérable de substances peut être emmagasiné dans les inclusions de corps hydrophiles polymérisés.

C'est pourquoi on cite par la suite un nombre considérable de substances possibles comme agents anticonceptionnels (page 5, lignes 15 à 32) ; la présence d'antibiotiques, la présence possible de substances permettant l'acclimatation dans l'utérus (page 4, lignes 17 à 20 et 22 à 35) ; la présence de produits analgésiques (page 4, lignes 21-22), l'exemple donné en page 11 est relatif à des inclusions hydrophiles dans lesquelles on a emmagasiné des agents chimiques et des agents d'acclimatation. La nature de l'agent chimique ou de l'agent d'acclimatation n'est pas indiquée.

D'autre part on connaît par l'abrégé 71 802n publié dans la revue Chemical Abstracts, page 57 volume 83 n° 9 du 1er Septembre 1975 un effet bénéfique de l'acide ε-amino-caproïque relativement à l'hémorragie utérine résultant de la présence de dispositif intrautérin en silicone.

La présente invention a pour but de fournir un dispositif endo-utérin contraceptif et thérapeutique perfectionné aboutissant à de nouveaux effets contraceptifs et thérapeutiques par la combinaison de plusieurs constituants dont les effets individuels ne sont pas contrariés par la présence des autres constituants, c'est-à-dire sont au moins conservés, et sont pour certains accrus par un effet de synergie inattendu.

Tous ces effets techniques inattendus sont obtenus selon la présente invention par la réalisation d'un dispositif endo-utérin contraceptif et thérapeutique comprenant du cuivre ainsi que de la progestérone naturelle et de l'acide amino-6-caproïque, ces deux dernières substances étant renfermées dans un matériau hydrophobe permettant leur diffusion en milieu aqueux, caractérisé en ce qu'il présente une forme de T dont les deux branches horizontales forment deux cavités indépendantes contenant respectivement la progestérone naturelle et l'acide amino-6-caproïque, et dont la jambe constitue un noyau d'argent sur lequel est enroulé un fil de cuivre.

Par cette combinaison selon l'invention des trois constituants essentiels, on aboutit de manière inattendue à un renforcement radial de l'effet contraceptif relativement à l'effet du métal isolé ou de la progestérone naturelle isolée. D'autre part, il apparaît aussi de manière inattendue que les effets individuels des constituants n'ont pas été contrariés par la présence des autres constituants.

On obtient en outre également en combinaison un effet thérapeutique dû à la progestérone consistant notamment en des modifications biochimiques de l'endomètre (baisse des phosphatases alcalines de la Béta-glycuronidase et lacticodéshydrogénase), une diminution de la synthèse endométriale de la prostaglandine d'où un effet thérapeutique dans le cas de dysménorrhées (règles douloureuses). On obtient également une diminution des flux menstruels du fait de la décidualisation de l'endomètre ce qui est intéressant dans le cas des hyperménorrhées.

On obtient aussi selon l'invention en combinaison une suppression des effets secondaires, notamment les saignements entre les règles, une normalisation du volume et du débit des règles, une suppression de l'effet systémique des médicaments tout en réduisant·le risque infectieux et un renforcement de l'efficacité contraceptive de manière tout à fait inattendue.

Suivant une autre caractéristique, la progestérone naturelle et/ou l'acide amino-6-caproïque est ou sont disposés individuellement dans du liquide siliconé entouré d'une membrane régulatrice de débit composée par exemple d'un copolymère d'éthylène et d'acétate de vinyle.

Ce dispositif est encore caractérisé par l'adjonction de sulfate de baryum dans sa masse pour renforcer la radio-opacité.

Selon un autre mode de réalisation, le dispositif selon cette invention présente une forme en delta définissant deux cavités fermées indépendantes contenant respectivement la progestérone naturelle et l'acide amino-6-caproïque.

Le mode de réalisation actuellement préféré de l'invention sera décrit en référence à la figure unique annexée, qui est donnée à titre d'exemple.

En référence à cette figure unique, un dispositif endo-utérin repéré généralement par le numéro de référence 1 a selon le mode de réalisation actuellement préféré une forme en T, c'est-à-dire comportant une jambe verticale 2 et deux branches horizontales 3, 4.

Ce dispositif endo-utérin 1 est de préférence réalisé en matériau hydrophobe permettant la diffusion de substances hydrophiles en milieu aqueux. Par exemple il est réalisé en polyéthylène. Ce dispositif endo-utérin est de préférence rendu radio-opaque par par exemple l'adjonction de sulfate de baryum dans sa masse.

De préférence, chaque branche 3, 4 du dispositif endo-utérin 1 est réalisée creuse, c'est-à-dire comporte une cavité respectivement 5, 6. La cavité 5 est naturellement indépendante de la cavité 6.

Selon l'invention, la cavité 5 renferme par exemple la progestérone naturelle représentée par le numéro de référence 7 tandis que l'autre cavité 6 renferme de l'acide amino-6-caproïque représenté par le numéro de référence 8.

D'autre part, le cuivre, ici de préférence sous forme d'un fil de cuivre 10 monté sur un noyau d'argent 12, est enroulé à l'extérieur de la jambe 2 du T.

La jambe 2 du T comporte à son extrémité libre une boucle caudale 14 permettant de fixer un ou plusieurs fils de nylon 16 qui servent au retrait du dispositif endo-utérin.

L'intérêt de renforcer le fil de cuivre par un noyau d'argent réside dans le fait que le noyau d'argent évite la fragmentation et prolonge la durée de vie du cuivre.

En réalisation pratique, la jambe 2 du T mesure 20 mm de long et est de forme arrondie, un diamètre de 1,5 mm et le poids du cuivre-argent est compris entre 200 et 400 mg, la surface du cuivre-argent étant comprise entre 200 à 400 mm². La libération du cuivre est alors de 50 à 150 microgrammes par jour.

Chaque branche 3, 4 du T présente une longueur de 15 mm environ et un diamètre de 1 mm. La cavité 5 renferme par exemple 52 mg de progestérone naturelle micropulvérisée tandis que la cavité 6 contient une quantité équivalente d'acide iso-amino-caproïque.

La progestérone naturelle micropulvérisée ou l'acide amino-iso-caproïque est de préférence placé dans du liquide siliconé qui peut être entouré d'une membrane régulatrice de débit, par exemple un copolymère d'éthylène et d'acétate de vinyle en améliorant le contrôle de la diffusion des produits jusqu'à la lumière utérine.

Dans le cas où la progestérone naturelle ou l'acide amino isocaproïque sont renfermés dans une telle membrane régulatrice de débit, les cavités 5, 6 n'ont pas besoin d'être physiquement séparées par une cloison 18 comme représenté, puisque cette membrane réalise cette séparation physique.

Ainsi, selon l'invention on obtient une délivrance de la progestérone s'étalant sur une période de 36 mois avec une libération de 65 microgrammes par jour, cette dose assurant la plus grande protection contraceptive ce qui correspond à une dose de 24 mg de progestérone délivrée en 1 an. La délivrance de l'acide amino-iso-caproïque s'effectue à un débit constant durant 795 jours temps de tolérance maximum pour le métal cuivre et pour la progestérone également délivrée en combinaison.

On doit encore noter que l'acide amino-iso-caproïque procure aussi un effet anti-allergique.

On comprend ainsi que l'invention comprend tous les moyens constituant des équivalents techniques des moyens décrits ainsi que leurs diverses combinaisons. Par exemple, le dispositif endo-utérin peut présenter diverses formes pratiques et être réalisé en divers matériaux sans pour autant sortir du cadre de l'invention.

En particulier, le dispositif endo-utérin selon l'invention, selon un autre mode de réalisation également préféré, présente une forme en delta (Δ), de préférence définissant deux cavités fermées indépendantes comme dans le cas du mode de réalisation en T, dans lesquelles on peut placer plus aisément les modules contenant respectivement la progestérone naturelle et l'acide amino-iso-caproïque que dans le cas des branches horizontales du T qui doivent être de faible dimension pour conserver une grande souplesse.

**Revendications**

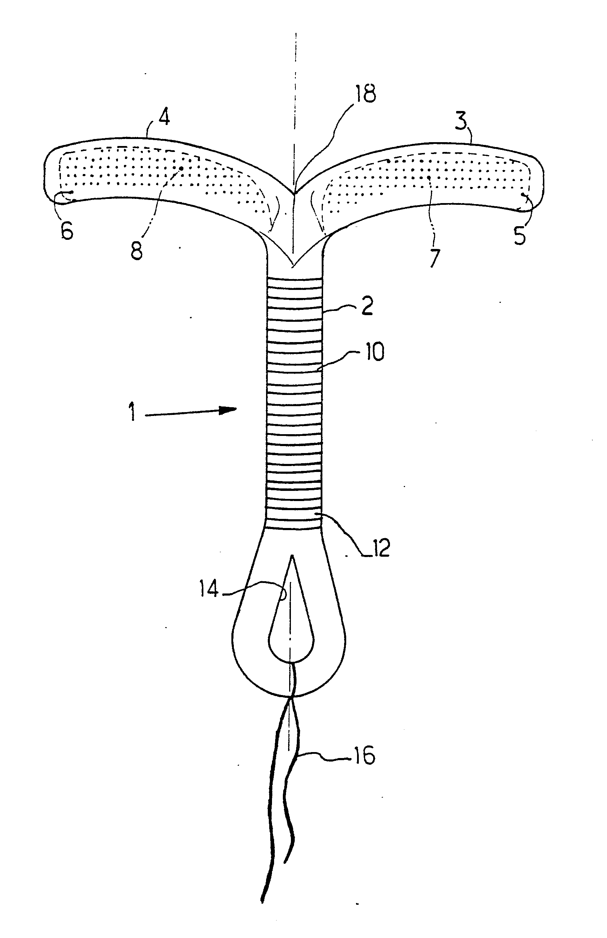

1. Dispositif endo-utérin contraceptif et thérapeutique comprenant du cuivre ainsi que de la progestérone naturelle et de l'acide amino 6 caproïque, ces deux dernières substances étant renfermées dans un matériau hydrophobe permettant leur diffusion en milieu aqueux, caractérisé en ce qu'il présente une forme de T selon la figure 1/1 dont les deux branches horizontales (3, 4) forment deux cavités indépendantes (5, 6) contenant respectivement la progestérone naturelle (7) et l'acide amino-6-caproïque (8), et dont la jambe (2) constitue un noyau d'argent (12) sur lequel est enroulé un fil de cuivre (10).

2. Dispositif selon la revendication 1, caractérisé en ce que la progestérone naturelle et/ou l'acide amino-6-caproïque est ou sont disposés individuellement dans du liquide siliconé entouré d'une membrane régulatrice de débit composée par exemple d'un copolymère d'éthylène et d'acétate de vinyle.

3. Dispositif selon la revendication 1 ou 2, caractérisé par l'adjonction de sulfate de baryum dans sa masse pour renforcer la radio-opacité.

4. Dispositif endo-utérin contraceptif et thérapeutique comprenant du cuivre ainsi que de la progestérone naturelle et de l'acide amino-6-caproïque, ces deux dernières substances étant renfermées dans un matériau hydrophobe permettant leur diffusion en milieu aqueux, caractérisé en ce qu'il présente une forme en delta (Δ) définissant deux cavités fermées indépendantes contenant respectivement les deux substances précitées.

5. Dispositif selon la revendication 4, caractérisé en ce que la progestérone naturelle et/ou l'acide amino-6-caproïque est ou sont disposés individuellement dans du liquide siliconé entouré d'une membrane régulatrice de débit composée par exemple d'un copolymère d'éthylène et d'acétate de vinyle.

6. Dispositif selon la revendication 4 ou 5, caractérisé par l'adjonction de sulfate de baryum dans sa masse pour renforcer la radio-opacité.

## Claims

1. Contraceptive and therapeutic endo-uterine device comprising copper as well as natural progesterone and amino-6-caproic acid, these two last substances being enclosed in a hydrophobic material allowing their diffusion in an aqueous medium, characterized in that it exhibits a T-shape according to Figure 1/1 both horizontal arms (3, 4) of which form two independent cavities (5, 6) containing the natural progesterone (7) and the amino-6-caproic acid (8), respectively and the leg (2) of which constitutes a silver core (12) on which is wound a copper wire (10).

2. Device according to claim 1, characterized in that the natural progesterone and/or the amino-6-caproic acid is or are individually disposed in a silicone-containing liquid surrounded by a flow-rate controlling membrane consisting for instance of a copolymer of ethylene and vinyl acetate.

3. Device according to claim 1 or 2, characterized by the addition of barium sulfate into its body for reinforcing the radio-opacity.

4. Contraceptive and therapeutic endo-uterine device comprising copper as well as natural progesterone and amino-6-caproic acid, these two latter substances being enclosed into a hydrophobic material allowing their diffusion in an aqueous medium, characterized in that it exhibits a delta-shape (Δ) defining two independent closed cavities containing both aforesaid substances, respectively.

5. Device according to claim 4, characterized in that the natural progesterone and/or the amino-6-caproic acid is or are individually disposed in a silicone-containing liquid surrounded by a flow-rate controlling membrane consisting for instance of a copolymer of ethylene and vinyl acetate.

6. Device according to claim 4 or 5, characterized by the addition of barium sulfate into its body for reinforcing the radio-opacity.

## Patentansprüche

1. Schwangerschaftverhütende und therapeutische Endouterinvorrichtung mit Kupfer sowie natürlichem Progesteron und Amino-6-Kapronsäure, wobei diese beiden letzteren Stoffe in einem deren Diffusion in einem wässerigen Medium gestattenden hydrophoben Material eingeschlossen sind, dadurch gekennzeichnet, dass sie eine T-Gestalt gemäss Figur 1/1 aufweist, deren beide waagerechte Schenkel (3, 4) zwei unabhängige, das natürliche Progesteron (7) und die Amino-6-Kapronsäure (8) enthaltende Aushöhlungen (5, 6) bildet und deren Bein (2) einen Silberkern (12) auf welchem ein Kupferdraht (10) gewickelt ist, bildet.

2. Vorrichtung nach Anspruch 1, dadurch gekennzeichnet, dass das natürliche Progesteron und/oder die Amino-6-Kapronsäure sich einzeln in einer silikonhaltigen Flüssigkeit befindet bzw. befinden, welche von einer die Durchsatzmenge regelnden, z. B. aus einem Copolymer von Äthylen und Vinylacetat bestehenden Membran umgeben ist.

3. Vorrichtung nach Anspruch 1 oder 2, gekennzeichnet durch den Zusatz von Bariumsulfat in ihrer Masse, um die Radio-Opazität zu verstärken.

4. Schwangerschaftverhütende und therapeutische Endouterinvorrichtung mit Kupfer sowie natürlichem Progesteron und Amino-6-Kapronsäure, wobei diese beiden letzteren Substanzen in einem deren Diffusion in wässerigem Medium gestattenden hydrophoben Material eingeschlossen sind, dadurch gekennzeichnet, dass sie eine Deltagestalt (Δ) aufweist, welche zwei geschlossene unabhängige jeweils die beiden vorgenannten Substanzen enthaltende Hohlräume bildet.

5. Vorrichtung nach Anspruch 4, dadurch gekennzeichnet, dass das natürliche Progesteron und/oder die Amino-6-Kapronsäure sich einzeln in einer silikonhaltige Flüssigkeit befindet bzw. befinden, welche von einer die Durchsatzleistung regelnden, z. B. aus einem Copolymer von Äthylen und Vinylacetat bestehenden Membran umgeben ist.

6. Vorrichtung nach Anspruch 4 oder 5, gekennzeichnet durch den Zusatz von Bariumsulfat in ihrer Masse, um die Radio-Opazität zu verstärken.